# EUROPEAN PATENT APPLICATION

(11) **EP 1 110 568 A2**
(43) Date of publication of application: **27.06.2001**
(21) Application number: 00127896.9
(22) Date of filing: 20.12.2000
(51) Int. Cl.: A61M 5/145

(54) **Syringe for infusing drugs**

(30) Priority: 21.12.1999 IT TO991129
(71) Applicant: CANE' S.r.l., 10090 Rivoli (TO) (IT)
(72) Inventor: Cane, Mario, 10095 Collegno (IT)
(74) Representative: Robba, Pierpaolo

(57) **Abstract**

A syringe for infusing drugs, comprising a barrel (2) with non-circular cross section in which a plunger (10; 10') slides, said cross section of the barrel (2) comprising a substantially quadrangular central portion (2a) and two half-circular side portions (2b).

## Description

The present invention relates to a syringe for infusing drugs, more particularly to a syringe with optimised cross-sectional shape, to be used jointly with a drug infusion apparatus.

Syringes for infusing liquid drugs, to be coupled with apparatuses capable of applying a slow and progressive thrust onto the syringe plunger through a slide, are known since a long time. Said apparatuses are to be used for long periods and thus they must ensure portability by the user with a minimum bulk.

One such apparatus is disclosed in Italian patent application T092A000561 in the name of the Applicant. The drug infusion apparatus according to the above patent application comprise a thrust member onto which the plunger of the syringe previously filled with drug is directly mounted. To this aim the syringe is equipped with a plunger having a removable stem that is used for drug intake and subsequently removed.

By removing the syringe stem, the size of the whole of the apparatus and the syringe is reduced, the portability is improved and the therapy can be carried out while leaving a greater freedom of movement to the user.

However, when the therapy requires the use of syringes intended for administering considerable drug amounts, the size of the whole of the apparatus and the syringe is cumbersome and, therefore, the whole of the apparatus and the syringe is not comfortable for portable use.

Even if syringes with non circular cross-section have been proposed in the past (see for instance US design patent D 403,761), the problem of how to optimise the size of a syringe in respect of the amount of drug to be contained and of its use with an infusion apparatus seems to have not been dealt with.

Thus, a first object of the invention is to provide a syringe with non-circular cross-section, the size of which is optimised in respect of the amount of drug to be contained and of the infusion apparatus.

Another problem the present invention aims to solve is to provide a syringe equipped with a plunger having a high tightness against liquid leakage.

The above and other objects of the invention are achieved by the syringe for infusing drugs as claimed in the appended claims.

The above objects will become more apparent from the detailed description of a preferred embodiment of the invention, with particular reference to the accompanying drawings, in which:
- Fig. 1 is a schematical view of the cross section of the barrel of the syringe according to the invention;
- Fig. 2 is a perspective view of the syringe according to the invention;
- Fig. 3 is a cross-sectional side view of the syringe shown in Fig. 2;
- Fig 4 is a plan view of the syringe shown in Fig. 2;
- Fig 5 is a plan view of the stem-guiding block of the syringe shown in Fig. 2;
- Fig. 6a shows a detail of the syringe according to the invention;
- Fig. 6b shows a detail of the syringe according to a second embodiment of the invention;
   Fig. 7 is a cross-sectional partial side view of an infusion apparatus equipped with a syringe according to the invention; and
   Fig. 8 is a partial side view of a syringe according to a variant embodiment of the invention.

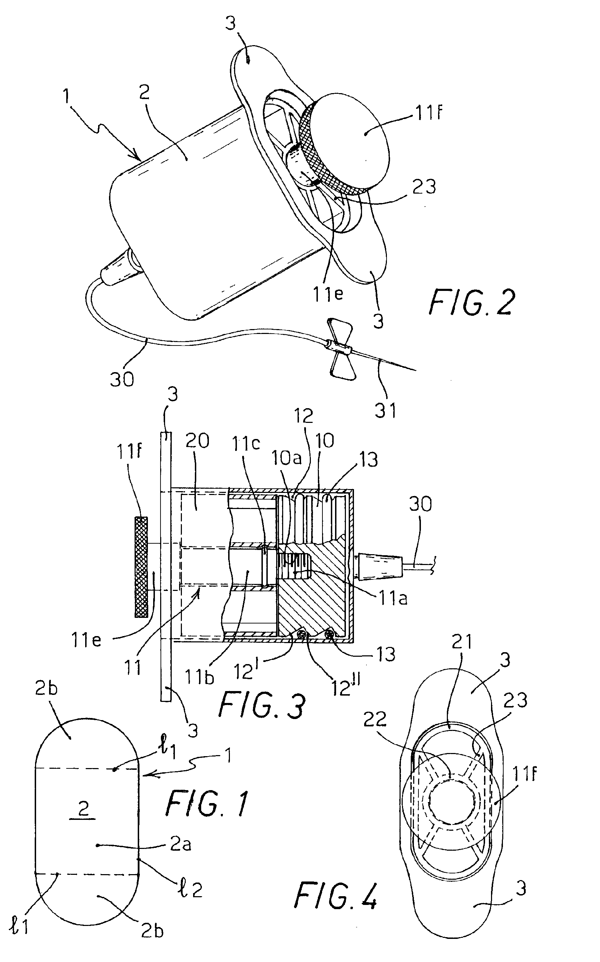

Referring to Fig. 1, the cross section of the barrel of a drug-infusing syringe 1 according to the invention is schematically shown.

Syringe 1 comprises a barrel 2 with non-circular cross section, having a substantially square central portion 2a with sides 1₁, 1₂ and two half-circular side portions 2b connected in correspondence of sides 1₁.

In the preferred embodiment, sides 1₁, 1₂ have the same lengths and the diametres of side portions 2b are equal to side 1₁ of central portion 2a. However, syringes can be produced where sides 1₁, 1₂ are different and the side portions are curved, but not half-circular.

Through the syringe according to the preferred embodiment of the invention, the external size of syringe 1 is optimised with respect to the amount of liquid to be injected and to the size of the infusion apparatus, without impairing the tightness of the syringe in respect of drug leakage during infusion.

The syringe barrel does not project laterally of the infusion apparatus, thereby avoiding uncomfortable bulks to the user, and it has a reduced length so that the whole of the syringe and the apparatus has a reduced size.

In this respect, it is to be appreciated that a syringe with circular cross section of diameter 1₁ has a length more than twice the length of the syringe of the preferred embodiment of the invention, with a square central portion of size 1₁, for the same amount of drug contained.

Referring to Figs. 2 to 4, syringe 1 is equipped with a plunger 10 and is connected through a flexible cannula 30 with a needle 31 suitable for being inserted under the patient's skin for parenteral injection of programmed drug doses.

Barrel 2 of syringe 1 has oppositely directed fins 3 to make the hold of syringe 1 during drug intake easier.

Plunger 10, made of plastics, has a threaded seat 10a into which the correspondingly threaded end 11a of a removable stem 11 is screwed. Stem 11 is used to move plunger 10 to intake the drug and then it is removed to allow mounting the filled syringe 1 onto an infusion apparatus.

To ensure tightness against liquid leakage during infusion, plunger 10 has, on its side wall, two rings 13 of natural or synthetic rubber or other resilient material, housed in peripheral grooves 12.

Each groove 12 has a frusto-conical surface 12' inclined towards the plunger centre in the direction of the forward movement of plunger 10 during infusion, and an abutment 12" for resilient ring 13.

Ring 13, because of the pressure of the liquid during infusion, is pushed rearwards with respect to the direction of the forward movement of plunger 10 and it expands while following the inclined profile of frusto-conical surface 12', thereby increasing its sealing action against the walls of syringe 1.

In the preferred embodiment of the invention, two grooves 12 and two rings 13 have been provided; yet, depending on the requirements of tightness against leakage, said number might be increased or decreased.

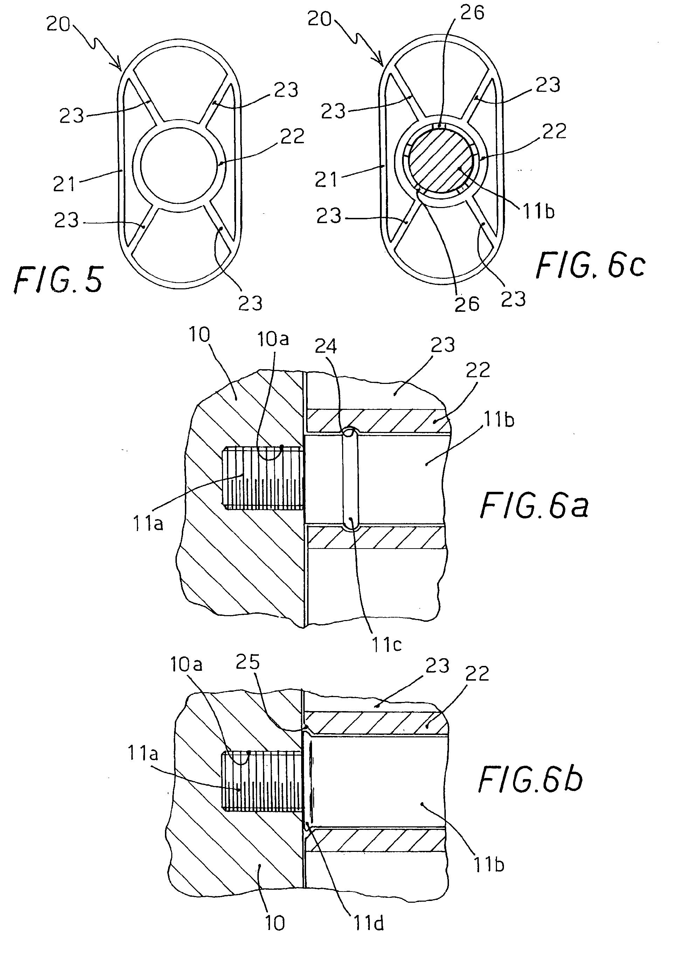

To avoid lateral oscillations of stem 11 while the liquid is being intaken, a plastic block 20, having the same cross section as the syringe barrel, is arranged between stem 11 and plunger 10 so as to guide stem 11. Referring to Fig. 5, said block 20 has a wall 21 to which a cylindrical sleeve 22, where stem 11 passes, is joined through spokes 23.

Figs. 6a and 6b show two alternative systems for securing block 20 to stem 11.

Fig. 6a shows a first system, consisting of a ring shaped protuberance 11c formed on body 11b of stem 11 and fitting into a corresponding circumferential seat 24 formed on cylindrical sleeve 22 of block 20.

Fig. 6b shows the second securing system, consisting of a protuberance 11d formed at the end of body 11b of stem 11 so as to give rise to a portion of enlarged diameter. That portion interferes with a corresponding circumferential taper 25, formed at the end of cylindrical sleeve 22 of block 20 so as to maintain the rotation capability of stem 11 when it is to be unscrewed from plunger 10.

As shown in Fig. 3, stem 11 is conveniently equipped with an enlarged portion 11e abutting against block 20 to avoid excessive screwing. Advantageously, stem 11 has a head 11f that is knurled to improve grip during screwing and unscrewing of said stem 11 into and from plunger 10.

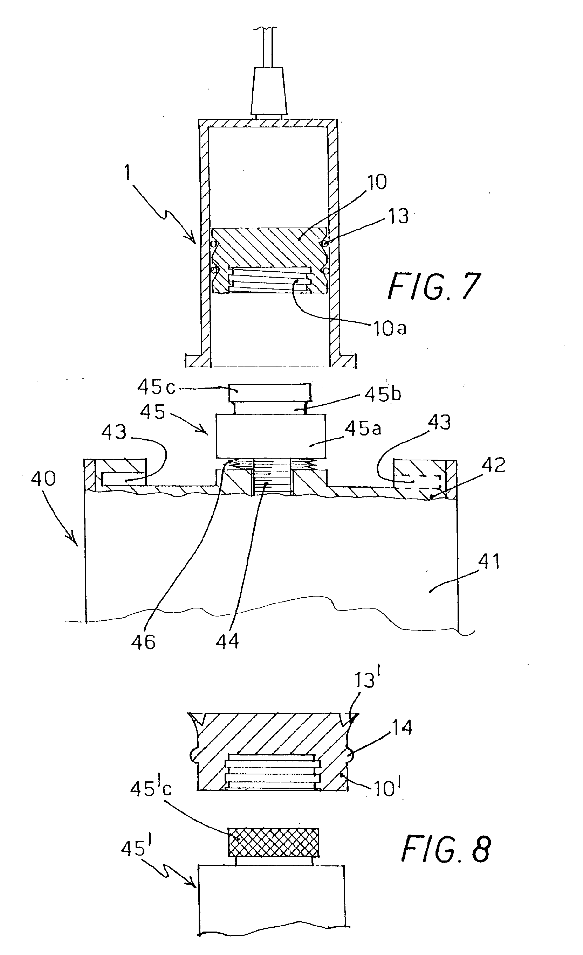

Fig. 7 schematically shows the head of an infusion apparatus 40 comprising a container 41 closed though a cap 42 onto which drug containing syringe 1 is mounted.

In order to secure syringe 1 onto apparatus 40, use is made of fins 3 that fit into corresponding grooves 43 formed on two opposite sides of cap 42.

Mounting of syringe 1 is obtained by introducing the syringe with fins 3 arranged transversally to the major dimension of apparatus 40 and by rotating the syringe by 90° so as to introduce fins 3 into grooves 43.

A threaded shaft 44 rotated by an electric drive, e.g. a geared motor, acts upon plunger 10 of syringe 1.

An adapter 45, intended to be inserted into threaded seat 10a formed in plunger 10, is secured to the end of threaded shaft 44 coming out from apparatus 40.

Adapter 45 comprises a cylindrical body having a first hollow portion 45a, a second, intermediate portion 45b and a third portion 45c, of lesser diameter than portion 45a. Adapter 45 is to retain plunger 10 during drug infusion.

Advantageously, adapter 45 has an extensible sleeve 46 fastened internally of the first hollow portion 45a, the other sleeve end being secured to cap 42 so as to isolate threaded shaft 44, and consequently the inside of apparatus 40, from the outside so as to prevent liquid leaking out of syringe 1 from damaging the internal components of the apparatus.

When, for any reason, the apparatus with the syringe containing the drug is at a level of some ten centimetres above the needle inserted under the skin, a free flow of the drug into the patient can occur.

Such a phenomenon, caused by a sudden pressure fall occurring in the syringe barrel, is allowed by the thrust member becoming detached form the plunger. Thus a free flow of liquid can be created, with a consequent drug overdose than can even seriously damage the patient.

To overcome that drawback, in a variant embodiment of the invention, shown in Fig. 8, plunger 10' is made of natural or synthetic rubber or other resilient material, and adapter 45' has head 45'c that is made rough, for instance though a knurling, so as to increase its friction coefficient and firmly secure plunger 10'.

In order to ensure tightness against leakage of the liquid, plunger 10' has a circumferential rim 13' on its edge facing the drug and a ring seal 14 on its side wall. Rim 13' becomes deformed under the action of the liquid pressure during infusion so as to expand and to adhere to the walls of syringe 1.

## Claims

1. A syringe for infusing drugs, comprising a barrel (2) with non-circular cross section in which a plunger (10; 10') slides, characterised in that the cross section of said barrel (2) comprises a substantially quadrangular central portion (2a) and two curved side portions (2b).

2. A syringe as claimed in claim 1, characterised in that the cross section of said barrel (2) has a square central portion (2a) and two half-circular side portions (2b).

3. A syringe as claimed in claim 1 or 2, characterised in that said plunger (10; 10') comprises resilient means (13; 13') that can become deformed under the action of the fluid pressure to increase tightness against leakage of liquid during infusion.

4. A syringe as claimed in claim 3, characterised in that said deformable resilient means comprise at least one ring (13) of natural or synthetic rubber housed within a corresponding frusto-conical peripheral groove (12) formed on said plunger (10).

5. A syringe as claimed in claim 4, characterised in that said plunger (10) is made of plastics.

6. A syringe as claimed in claim 4 or 5, characterised in that said at least one groove (12) has an edge (12') inclined towards the plunger centre in the direction of the forward movement of the plunger (10), so that said resilient ring (13) housed in the groove is made to expand against the wall of the syringe (1) by the liquid pressure during the forward movement of the plunger (10).

7. A syringe as claimed in claim 3, characterised in that said plunger (10') is made of a resilient material such as natural or synthetic rubber, and in that said deformable means comprise a circumferential rim (13') formed on the edge of plunger (10') facing the liquid.

8. A syringe as claimed in any preceding claim, characterised in that it further comprises a stem (11) for moving the plunger (10; 10'), and in that said stem (11) is removable from said plunger (10; 10').

9. A syringe as claimed in claim 8, characterised in that it comprises a block (20) of plastics arranged between the stem (11) and the plunger (10; 10') so as to guide said stem (11) during drug intake.

10. A syringe as claimed in claim 9, characterised in that said block (20) comprises means (24; 25) for securing said stem (11) inside the block.
